# DEMANDE DE BREVET EUROPEEN

(11) **EP 1 013 266 A1**
(43) Date de publication de la demande: **28.06.2000**
(21) Numéro de dépôt: 99403110.2
(22) Date de dépôt: 10.12.1999
(51) Int. Cl.: A61K 7/48

(54) **Utilisation de composés N-acylés d'aminoacides comme agent de texture**

(30) Priorité: 22.12.1998 FR 9816239
(71) Demandeur: SOCIETE D'EXPLOITATION DE PRODUITS POUR LES INDUSTRIES CHIMIQUES-SEPPIC, F-75321 Paris Cédex 07 (FR)
(72) Inventeur: Michel, Nelly, 94700 Maisons Alfort (FR); Berger, Christian, 69130 Ecully (FR)
(74) Mandataire: Conan, Philippe Claude

(57) **Abrégé**

Utilisation d'au moins un composé de formule (I): 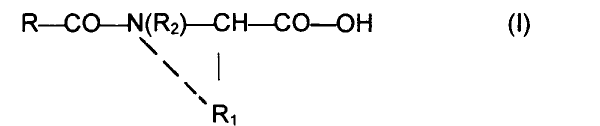 dans laquelle R représente la chaîne caractérisante d'un acide gras, saturé ou insaturé, linéaire ou ramifié, comportant de 3 à 30 atomes de carbone, R₁ ou 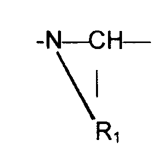 représente la chaîne ou le radical cyclique caractérisant d'un aminoacide, R₂ représente un atome d'hydrogène ou un radical alkyle comportant de 1 à 3 atomes de carbone, ou de leurs sels topiquement acceptables comme agent de texture, étant entendu que l'aminoacide caractérisé par R₁, n'est, ni la lysine, ni l'hydroxylysine, ni l'arginine. Nouvelles compositions. Application en cosmétique.

## Description

La présente invention a pour objet une nouvelle utilisation de dérivés N-acylés d'aminoacides Ces composés, comme par exemple ceux décrits dans les demandes internationales de brevet publiées sous les numéros WO92/20647, WO92/21318, WO94/26694, WO94/27561 et WO 98/09611 sont, en raison notamment de leur activité anti-microbienne et anti-élastasique, utilisés comme actifs dans les formulations à usage topiques. De telles formulations, qu'elles soient cosmétiques, dermopharmaceutiques ou pharmaceutiques, destinées aux soins de la peau ou des muqueuses, doivent notamment posséder les propriétés suivantes :

Avant l'application sur la peau, elles doivent être facilement préhensibles ;
au moment de l'application sur la peau ou les muqueuses, elle doivent s'étaler facilement, pénétrer rapidement, être fondantes tout en procurant des sensations de fraîcheur et de douceur, et sans être perçues comme grasses ou huileuses ;
après l'application sur la peau ou les muqueuses, elles ne doivent ni engendrer de brillance de la peau, ni d'odeurs désagréables, ni paraître collantes ; par contre, les sensations de douceur et de fraîcheur résultant de leur application, doivent persister.

Or, pour obtenir un profil sensoriel satisfaisant d'une formulation, il est souvent nécessaire d'y inclure des agents de texture, qui sont des composés ou des mélanges de composés susceptibles d'améliorer les caractéristiques rhéologiques et sensorielles de ladite formulation. La demanderesse a trouvé de façon inattendue, que les dérivés N-acylés d'aminoacides pouvaient être utilisés efficacement comme agent de texture de formulations cosmétiques, dermo-pharmaceutiques ou pharmaceutiques C'est pourquoi l'invention a pour objet l'utilisation d'au moins un composé de formule (I): 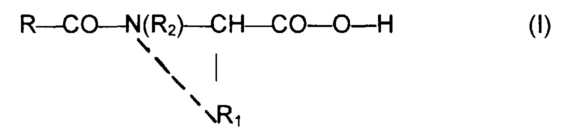 ou ses sels topiquement acceptables,
dans laquelle R représente la chaîne caractérisante d'un acide gras, saturé ou insaturé, linéaire ou ramifié, comportant de 3 à 30 atomes de carbone,
R₁ ou 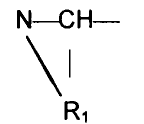 représente la chaîne ou le radical cyclique caractérisant l'aminoacide et R₂ représente un atome d'hydrogène ou un radical alkyle comportant de 1 à 3 atomes de carbone, comme agent de texture, étant entendu que l'aminoacide caractérisé par R₁ n'est ni la lysine, ni l'hydroxylysine, ni l'arginine.

Par sel topiquement acceptable, on entend tout sel de l'acide de formule (I) biologiquement acceptable pour la peau et/ou les muqueuses, c'est à dire tout sel pouvant notamment régler le pH de la composition à une valeur comprise entre 3 et 8 et de préférence environ égale à 5, ce qui est un pH voisin de celui de la peau. Il peut s'agir notamment de sels alcalins tels que les sels de sodium, de potassium ou de lithium, de sels alcalino-terreux tels que les sels de calcium, de magnésium ou de strontium; il peut aussi s'agir de sels métalliques tels que les sels divalents de zinc, de cuivre ou de manganèse ou encore les sels trivalents de fer, de lantane, de cérium ou d'aluminium. Le composé de formule (I) présent dans la composition objet de la présente invention, peut être sous forme d'acide libre ou sous forme partiellement ou totalement salifiée.

L'expression "chaîne caractérisante" utilisée dans le cadre de la présente demande de brevet, désigne la chaîne principale de l'acide gras ou de l'acide aminé considéré. Ainsi, pour un acide gras répondant à la formule générale R-COOH, la chaîne caractérisante sera la chaîne représentée par R. Le radical R représente notamment un radical comportant de 5 à 22 atomes de carbone choisi parmi les radicaux pentyle, hexyle, heptyle, octyle, nonyle, décyle, undécyle, dodécyle, tridécyle, tétradécyle, pentadécyle, hexadécyle, heptadécyle, octadécyle, nonadécyle, eicosyle, uneicosyle, docosyle, heptadécènyle, eicosènyle, uneicosènyle, docosènyle ou heptadécadiènyle ou décènyle.

L'invention a plus particulièrement pour objet l'utilisation telle que définie précédemment, pour laquelle, dans la formule (I), le fragment R-CO comporte de 7 à 22 atomes de carbone et représente notamment l'un des radicaux hexanoyle, heptanoyle, octanoyle (capryloyle), décanoyle (caproyle), undécylènoyle, dodécanoyle (lauroyle), tétradécanoyle (myristyle), hexadécanoyle (palmitoyle), octadécanoyle (stéaryle), eicosanoyle (arachidoyle), docosanoyle (behènoyle), octodécènoyle (oléyle), éicosènoyle (gadoloyle), docosènoyle (érucyle) ou octadécadiènoyle (linolènoyle). Dans une première variante préférée de la présente invention, dans la formule (I), le fragment R-CO (I) comporte de 12 à 22 atomes de carbone et plus particulièrement, de 16 à 22 atomes de carbone.

Pour un aminoacide représenté par la formule générale :

HN(R₂)-CHR₁-COOH,

il sera caractérisé par la définition de R₁ ; pour un aminoacide représenté par la formule générale : 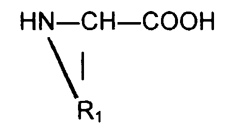 il sera caractérisé par la définition du radical cyclique :

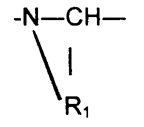

Dans le cadre de la présente invention, la chaîne ou le radical cyclique caractérisant l'aminoacide sont choisis parmi ceux de la glycine, de l'alanine, de la sérine, de l'acide aspartique, de l'acide glutamique, de la valine, de la thréonine, de la proline, de la leucine, de la phénylalanine, de l'isoleucine, de l'histidine, de la tyrosine, du tryptophane, de l'asparagine, de la cystéine, de la cystine, de la méthionine, de l'hydroxyproline, de l'ornithine et de la sarcosine. L'invention a plus particulièrement pour objet l'utilisation telle que décrite précédemment pour laquelle, dans la formule (I), la chaîne ou le radical cylique caractérisant l'aminoacide sont choisis parmi ceux de la glycine, de la proline, de l'acide glutamique ou de la sarcosine.

Par au moins un composé de formule (I), on indique que l'invention peut être mise en oeuvre avec un ou plusieurs de ces composés. Par plusieurs de ces composés, on entend :
soit un mélange de composés de formules (I), résultant de la condensation d'un seul aminoacide avec un mélange d'acides gras, et plus particulièrement un mélange d'acides gras d'origine naturelle, soit un mélange de composés de formules (I), résultant de la condensation d'un mélange d'aminoacides avec un seul acide gras, soit un mélange de composés de formules (I), résultant de la condensation d'un mélange d'aminoacides avec un mélange d'acides gras, et plus particulièrement avec un mélange d'acides gras d'origine naturelle. Les mélanges d'acides gras d'origine naturelle, susceptibles d'être utilisés avantageusement pour la préparation des composés de formules (I), sont notamment ceux dérivés de l'huile de coprah, de l'huile de palmiste, de l'huile de palme, de l'huile de soja, de l'huile de colza, du suif de boeuf, de l'huile de spermaceti, de l'huile de hareng ou de l'huile de ricin.

Selon un aspect tout particulier de la présente invention, celle-ci a pour objet l'utilisation telle décrite précédemment, d'un mélange comprenant au moins un composé de formule (la) correspondant à la formule (I), dans laquelle le radical cyclique caractérisant l'aminoacide, est celui de la proline et au moins un composé de formule (Ib) correspondant à la formule (I) dans laquelle la chaîne caractérisante de l'aminoacide est celle de l'acide glutamique et éventuellement au moins un composé de formule (Ic), correspondant à la formule (I) dans laquelle R₂ représente un radical méthyle et R₁ la chaîne caractérisante de la sarcosine; comme exemple d'une telle utilisation ,on peut citer l'utilisation du mélange comprenant de la N-palmitoyl proline et de l'acide N-palmitoyl glutamique ou leurs sels de sodium ou de magnésium ou l'utilisation du mélange comprenant de la N-palmitoyl proline, de l'acide N-palmitoyl glutamique et de la N-palmitoyl sarcosine ou leurs sels de sodium ou de magésium.

Selon un autre aspect tout particulier de la présente invention, celle-ci a pour objet, l'utilisation telle que décrite précédemment, d'une composition comprenant de la N-cocoyl glycine ou un de ses sels topiquement acceptables.
Les composés de formule (I) sont obtenus par les méthodes connues de l'homme du métier et décrites notamment dans les demandes internationales de brevet publiées sous les numéros WO 92/20647, WO 92/21318, WO 94/26694, WO 94/27561 et WO 98/09611.
L'invention a aussi pour objet une composition (A) comprenant :
a) de 10% à 95% en poids et, plus particulièrement, de 60% à 80% en poids, d'au moins un composé de formule (I): 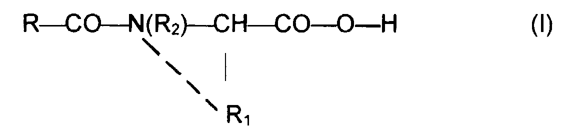 dans laquelle R représente la chaîne caractérisante d'un acide gras, saturé ou insaturé, linéaire ou ramifié, comportant de 3 à 30 atomes de carbone, 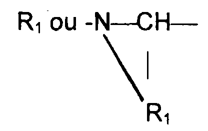 représente la chaîne ou le radical cyclique caractérisant l'aminoacide et R₂ représente un atome d'hydrogène ou un radical alkyle comportant de 1 à 3 atomes de carbone, étant entendu que l'aminoacide caractérisé par R₁ n'est ni la lysine, ni l'hydroxylysine, ni l'arginine, et
b) de 90% à 5% d'au moins un composé de formule (II)

   R-CO-OH

   ou un de leurs sels topiquement acceptables,

L'invention a plus particulièrement pour objet, une composition (A) telle définie ci-dessus, pour la quelle le radical R comporte de 16 à 22 atomes de carbones, et plus particulièrement, de 16 à 18 atomes de carbone. Selon un aspect tout particulier de la présente invention, celle-ci a pour objet la composition (A) telle que définie précédemment, comprenant :
de 30% à 50% en poids de palmitoyl proline,
de 5% à 25% en poids d'acide N-palmitoyl glutamique,
de 0% à 25% en poids de N-palmitoyl sarcosine et
d'environ 5% à environ 40% d'acide palmitique.
Selon un autre aspect tout particulier de la présente invention, celle-ci a pour objet la composition (A) telle que définie précédemment, comprenant :
de 30% à 70% en poids de cocoyl glycine,
de 0% à 25% en poids de N-cocoyl sarcosine et
d'environ 5% à environ 45% en poids, et plus particulièrement de 5% à environ 20% en poids du mélange d'acides gras issu de l'huile de coprah.
La présente invention a aussi pour objet, une formulation cosmétique, dermopharmaceutique ou pharmaceutique, comprenant comme agent de texture, une quantité efficace, d'au moins un composé de formule (telle que définie précédemment). Par quantité efficace, on entend dans le cadre de la présente invention une quantité représentant d' environ 0,1% à environ 5% en poids de ladite formulation.

La formulation cosmétique se présente habituellement sous forme d'une solution aqueuse, d'une solution alcoolique diluée, ou d'une émulsion simple ou multiple, telle qu'une émulsion eau dans huile (E/H), huile dans eau (H/E) ou eau dans huile dans eau (E/H/E), d'une dispersion de liposomes, ou d'aminosomes". On peut citer, par exemple les crèmes, les laits, les lotions, les émulsions solaires, les lingettes, les gels tels que les gels douches, les gels-crème, les huiles, les savons, les savons liquides, les syndets, les produits d'hygiène intime, les shampooings, les poudres de maquillage ou les mascaras.

L'invention a aussi pour objet, l'utilisation d'une composition (A) telle que définie précédemment pour préparer une formulation cosmétique, dermopharmaceutique ou pharmaceutique. Cette formulation est préparée seIon les méthodes connues de l'homme du métier qui sont appropriées au type de formulation recherché, en combinant la composition (A) avec les actifs cosmétiques et les excipients usuels.

Les composés de formule (I) sont compatibles avec la plupart des excipients connus, que ce soit par exemple avec les latex inverses tels que les composés commercialisés sous les noms SEPIGEL™ 305, SEPIGEL™ 501, SEPIGEL™ 502, SIMULGEL™ 600 ou SIMULGEL ™EG, ou avec les compositions commercialisées sous les noms MONTANOV™ 68, MONTANOV™ 14, MONTANOV™ 82, MONTANOV™ 202 ou SEPIPERL™ N et décrites, par exemple, dans les demandes de brevet publiées sous les numéros, W092/02778, WO95/13863, W096/37285, W098/22207 ou WO98/47610. Les exemples suivants illustrent l'invention sans toutefois la limiter.

### Exemple 1 : Profil sensoriel d'une composition selon l'invention

a) On prépare une composition comprenant environ 45% de palmitoyl proline, de 10% à 15% d'acide N-palmitoyl glutamique, de 10% à 15% de N-palmitoyl sarcosine et environ 30% d'acide palmitique.
b) on prépare un gel-crème (I) contenant comme agent de texture 3% de la composition préparée au paragraphe a) et, comme agent épaississant, 3,5% de SEPIGEL™ 305 et, un gel-crème (II) contenant comme agent épaississant 3,5 de SEPIGEL™ 305 et ne contenant pas d'agent de texture.
c) Le test sensoriel a été réalisé sur un panel de 15 personnes, ces personnes apprécièrent et comparèrent les qualités des deux gel-crèmes dans les trois situations suivantes :
   1) Appréciation de la prise du gel-crème avec les doigts en vue de l'étalement ultérieur sur la peau
      Le panel apprécia la facilité de prise
   2) Appréciation à l'application du gel-crème sur la peau
      Le panel apprécia la facilité d'étalement, le fondant, la sensation de fraîcheur, la douceur du produit, l'absence de sensation grasse du produit et la rapidité de pénétration.
   3) Appréciation après l'application
      Le panel apprécia l'odeur sur la peau, la brillance de la peau, l'aspect collant sur la peau, la douceur de la peau et la présence résiduelle.

Les résultats sont consignés dans le tableau suivant de la façon suivante :
- appréciations positives ou appréciations négatives lorsqu'au moins les deux tiers des membres sont du même avis :
- appréciation non différenciée (indiquée idem) lorsque moins des deux tiers des membres sont du même avis :
-

| | Gel-crème (I) invention | Gel-crème (II) Comparaison |
|---|---|---|
| Facilité de prise | Plus facile | Moins facile |
| facilité d'étalement | Plus facile | Moins facile |
| le fondant | Plus fondant | Moins fondant |
| Sensation de fraîcheur | Idem | Idem |
| Douceur | Plus doux | Moins doux |
| sensation grasse | Moins gras | Plus gras |
| pénétration. | Plus rapide | Moins rapide |
| l'odeur sur la peau | Idem | Idem |
| Brillance de la peau | Moins brillant | Plus brillant |
| Collant sur la peau | Idem | Idem |
| Douceur de la peau | Plus douce | Moins douce |
| Rémanence | Plus de rémanence | Moins de rémanence |

Ces résultats montrent que la présence de composés de formule (I) dans une formulation cosmétique améliore nettement le profil sensoriel de ladite formulation.

### Exemples de formulation cosmétiques (Exemples 2 à 32)

En utilisant la composition préparée au paragraphe a) de l'exemple 1, (appelée par la suite composition A), on a préparé les formulations cosmétiques suivantes:

### Exemple 2 : Crème de soin

| | |
|---|---|
| Cyclométhicone | 10% |
| Composition A | 3% |
| SEPIGEL® 502 | 0,8% |
| MONTANOV®68 | 2% |
| alcool stéarylique | 1,5% |
| conservateur | 0,65% |
| Lysine | 0,025% |
| EDTA (sel disodique) | 0,05% |
| Gomme de xanthane | 0,2% |
| Glycérine | 3% |
| Eau | q.s.p. 100% |

### Exemple 3 : Crème de soin

| | |
|---|---|
| Cyclométhicone | 10% |
| Composition A | 3% |
| SEPIGEL® 502 | 0,8% |
| MONTANOV® 68 | 2% |
| Perfluoropolymethylisopropylether | 0,5% |
| alcool stéarylique | 1,5% |
| conservateur | 0,65% |
| Lysine | 0,025% |
| EDTA (sel disodique) | 0,05% |
| PEMULEN®TR | 0,2% |
| Glycérine | 3% |
| Eau | q.s.p. 100% |

### Exemple 4: Baume après-rasage

| FORMULE | | |
|---|---|---|
| A | Composition A | 3% |
| | SEPIGEL™502 | 1,5% |
| | Eau | q.s.p 100% |
| B | MICROPEARL™ M 100 | 5,0% |
| | SEPICIDE™ Cl | 0,50% |
| | Parfum | 0,20% |
| | éthanol 95° | 10,0% |

| MODE OPERATOIRE | | |
|---|---|---|
| Ajouter B dans A. | | |

### Exemple 5 : Emulsion satinée pour le corps

| FORMULE | | |
|---|---|---|
| A | SIMULSOL™ 165 | 5,0% |
| | LANOL™ 1688 | 8,50% |
| | beurre de Karité | 2% |
| | huile de paraffine | 6,5% |
| | LANOL™ 14M | 3% |
| | LANOL™ S | 0,6% |
| B | eau | 66,2% |
| C | MICROPEARL™ M 100 | 5% |
| D | Composition A | 3% |
| | SEPIGEL™ 502 | 3% |
| E | SEPICIDE™ Cl | 0,3% |
| | SEPICIDE™ HB | 0,5% |
| | MONTEINE™ CA | 1% |
| | Parfum | 0,20% |
| | acétate de vitamine E | 0,20% |
| | Sodium pyrolidinone carboylate | 1% (agent hydratant) |

| MODE OPERATOIRE | | |
|---|---|---|
| Ajouter C dans B, émulsionner B dans A à 70°C, puis ajouter D à 60°C puis E à 30°C. | | |

### Exemple 6 : Lait corporel

| FORMULE | | |
|---|---|---|
| A | SIMULSOL™ 165 | 5,0% |
| | LANOL™ 1688 | 12,0% |
| | LANOL™ 14M | 2,0% |
| | alcool cétylique | 0,3% |
| | SCHERCEMOL™ OP | 3% |
| B | eau | q.s.p. 100% |
| C | Composition A | 3% |
| | SEPIGEL™ 502 | 0,35% |
| D | SEPICIDE™ Cl | 0,2% |
| | SEPICIDE™ HB | 0,5% |
| | Parfum | 0,20% |

| MODE OPERATOIRE | | |
|---|---|---|
| Emulsionner B dans A vers 75°C ; ajouter C vers 60°C, puis D vers 30°C | | |

### Exemple 7 : crème H/E

| FORMULE | | |
|---|---|---|
| A | SIMULSOL™165 | 5,0% |
| | LANOL™1688 | 20,0% |
| | LANOL™ P | 1,0% |
| B | eau | q.s.p. 100% |
| C | Composition A | 3% |
| | SEPIGEL™ 502 | 2,5% |
| D | SEPICIDE™ Cl | 0,20% |
| | SEPICIDE™ HB | 0,30% |

| MODE OPERATOIRE | | |
|---|---|---|
| Introduire B dans A vers 75°C ; ajouter C vers 60°C, puis D vers 45°C. | | |

### Exemple 8 : gel solaire non gras

| FORMULE | | |
|---|---|---|
| A | Composition A | 3% |
| | SEPIGEL™ 502 | 0,8% |
| | Eau | 30% |
| B | SEPICIDE™ Cl | 0,20% |
| | SEPICIDE™ HB | 0,30% |
| | Parfum | 0,10% |
| C | colorant | q.s. |
| | Eau | 30% |
| D | MICROPEAR™ M 100 | 3,00% |
| | Eau | q.s.p 100% |
| E | huile de silicone | 2,0% |
| | PARSOL™ MCX | 5,00% |

| MODE OPERATOIRE | | |
|---|---|---|
| Introduire B dans A; ajouter C, puis D, puis E. | | |

### Exemple 9 : Lait solaire

| FORMULE | | |
|---|---|---|
| A | SEPIPERL™ N | 3,0% |
| | huile de sésame | 5,0% |
| | PARSOL™ MCX | 5,0% |
| | Carraghénane λ | 0,10% |
| B | eau | q.s.p.100% |
| C | Composition A | 3% |
| | SEPIGEL™ 502 | 0,8% |
| D | parfum | q.s. |
| | Conservateur | q.s. |

| MODE OPERATOIRE | | |
|---|---|---|
| Emulsionner B dans A à 75°C puis ajouter C vers 60°C, puis D vers 30°C et ajuster le pH si nécessaire. | | |

### Exemple 10 : Gel de massage

| FORMULE | | |
|---|---|---|
| A | Composition A | 3% |
| | SEPIGEL™ 501 | 3,5% |
| | Eau | 20,0% |
| B | colorant | 2 gouttes/100g |
| | Eau | q.s. |
| C | alcool | 10% |
| | Menthol | 0,10% |
| D | huile de silicone | 5,0% |

| MODE OPERATOIRE | | |
|---|---|---|
| Ajouter B dans A; puis ajouter au mélange, C puis D | | |

### Exemple 11 : gel soin de massage

| FORMULE | | |
|---|---|---|
| A | Composition A | 3% |
| | SEPIGEL™ 502 | 3,0% |
| | Eau | 30% |
| B | SEPICIDE™ Cl | 0,20% |
| | SEPICIDE™ HB | 0,30% |
| | Parfum | 0,05% |
| C | colorant | q.s. |
| | Eau | q.s.p 100% |
| D | MICROPEARL™ SQL | 5,00% |
| | LANOL™ 1688 | 2% |

| MODE OPERATOIRE | | |
|---|---|---|
| Préparer A; additionner B, puis C, puis D. | | |

### Exemple 12 : Gel coup d'éclat

| FORMULE | | |
|---|---|---|
| A | Composition A | 3% |
| | SEPIGEL™ 502 | 4% |
| | Eau | 30% |
| B | ELASTINE HPM | 5,0% |
| C | MICROPEARL™ M 100 | 3% |
| | Eau | 5% |
| D | SEPICIDE™ Cl | 0,2% |
| | SEPICIDE™ HB | 0,3% |
| | Parfum | 0,06% |
| | Sodium pyrolidinone carboxylate | 50% : 1% |
| | Eau | q.s.p. 100% |

| MODE OPERATOIRE | | |
|---|---|---|
| Préparer A; additionner B, puis C, puis D. | | |

### Exemple 13 : Lait corporel

| FORMULE | | |
|---|---|---|
| A | SEPIPERL™ N | 3,0% |
| | Triheptonate de glycerol | 10,0% |
| B | eau | q.s.p.100% |
| C | Composition A | 1,5% |
| | SEPIGEL™ 502 | 1,0% |
| D | parfum | q.s. |
| | Conservateur | q.s. |

| MODE OPERATOIRE | | |
|---|---|---|
| Fondre A à environ 75°C . Emulsionner B dans A à 75°C puis ajouter C vers 60°C, puis D | | |

### Exemple 14 : Emulsion démaquillante à l'huile d'amande douce

| FORMULE | |
|---|---|
| MONTANOV™ 68 | 5% |
| huile d'amandes douces | 5% |
| eau | q.s.p.100% |
| Composition A | 1 % |
| SEPIGEL™ 502 | 0,3% |
| Glycérine | 5% |
| Conservateur | 0,2% |
| Parfum | 03% |

### Exemple 15 : Crème hydratante pour peaux grasses

| FORMULE | |
|---|---|
| MONTANOV™ 68 | 5% |
| Cétylstéaryloctanoate | 8% |
| octyl palmitate | 2% |
| eau | q.s.p.100% |
| Composition A | 2% |
| SEPIGEL™ 502 | 0,6% |
| MICROPEARL™ M100 | 3,0% |
| Mucopolysaccharides | 5% |
| SEPICIDE™ HB | 0,8 |
| Parfum | 03% |

### Exemple 16: Baume après-rasage apaisant sans alcool

| FORMULE | |
|---|---|
| LANOL™ 99 | 2% |
| huile d'amandes douces | 0,5% |
| eau | q.s.p.100% |
| Composition A | 3% |
| SEPIGEL™ 501 | 3% |
| SEPICIDE™ HB | 0,3% |
| SEPICIDE™ Cl | 0,2% |
| Parfum | 0,4% |

### Exemple 17 : Crème aux AHA pour peaux sensibles

| FORMULE | |
|---|---|
| LANOL™ 99 | 2% |
| MONTANOV™ 68 | 5,0% |
| Eau | q.s.p.100% |
| Composition A | 0,1 à 5% |
| SEPIGEL™ 501 | 1,5% |
| acide gluconique | 1,50% |
| triéthylamine | 0,9% |
| SEPICIDE™ HB | 0,3% |
| SEPICIDE™ Cl | 0,2% |
| Parfum | 0,4% |

### Exemple 18 : Soin apaisant après-soleil

| FORMULE | |
|---|---|
| LANOL™ 99 | 10,0% |
| Eau | q.s.p.100% |
| Composition A | 3% |
| SEPIGEL™ 502 | 2,5% |
| SEPICIDE™ HB | 0,3% |
| SEPICIDE™ Cl | 0,2% |
| Parfum | 0,4% |
| Colorant | 0,03% |

### Exemple 19 : Lait démaquillant

| FORMULE | |
|---|---|
| SEPIPERL™ N | 3% |
| PRIMOL™ 352 | 8,0% |
| huile d'amandes douces | 2% |
| eau | q.s.p.100% |
| Composition A | 2% |
| SEPIGEL™ 502 | 0,8% |
| Conservateur | 0,2% |

### Exemple 20 : Lait corporel

| FORMULE | |
|---|---|
| SEPIPERL™ N | 3,5% |
| LANOL™ 37T | 8,0% |
| SOLAGUM™ L | 0,05% |
| Eau | q.s.p.100% |
| Benzophénone | 2,0% |
| diméthicone 350cPs | 0,05% |
| Composition A | 2% |
| SEPIGEL™ 502 | 0,8% |
| Conservateur | 0,2% |
| Parfum | 0,4% |

### Exemple 21 : émulsion fluide à pH alcalin

| | |
|---|---|
| MARCOL™ 82 | 5,0% |
| NaOH | 10,0% |
| Eau | q.s.p.100% |
| Composition A | 4% |
| SEPIGEL™ 502 | 1,5% |

### Exemple 22 : Fond de teint fluide

| FORMULE | |
|---|---|
| SIMULSOL™ 165 | 5,0% |
| LANOL™ 84D | 8,0% |
| LANOL™ 99 | 5,0% |
| Eau | q.s.p.100% |
| pigments et charges minérales | 10,0% |
| Composition A | 3% |
| SEPIGEL™ 502 | 1,2% |
| Conservateur | 0,2% |
| Parfum | 0,4% |

### Exemple 23 : Lait solaire

| FORMULE | |
|---|---|
| SEPIPERL™ N | 3,5% |
| LANOL™ 37T | 10,0% |
| PARSOL NOX™ | 5,0% |
| EUSOLEX™ 4360 | 2,0% |
| Eau | q.s.p.100% |
| Composition A | 3% |
| SEPIGEL™ 502 | 1,8% |
| Conservateur | 0,2% |
| Parfum | 0,4% |

### Exemple 24 : Gel contour des yeux

| FORMULE | |
|---|---|
| Composition A | 1% |
| SEPIGEL™ 502 | 2% |
| Parfum | 0,06% |
| Sodium pyrrolidinonecarboxylate | 0,2% |
| DOW CORNING™ 245 Fluid | 2,0% |
| Eau | q.s.p. 100% |

### Exemple 25 : composition de soin non rincée

| FORMULE | |
|---|---|
| Composition A | 3% |
| SEPIGEL™ 502 | 1,5% |
| Parfum | q.s |
| Conservateur | q.s. |
| DOW CORNING™ X2 8360 | 5,0% |
| DOW CORNING™ Q2 1401 | 15,% |
| Eau | q.s.p. 100% |

### Exemple 26 : gel amincissant

| | |
|---|---|
| Composition A | 5% |
| SEPIGEL™ 501 | 5% |
| Ethanol | 30 % |
| Menthol | 0,1 % |
| Caféine | 2,5 % |
| extrait de ruscus | 2 % |
| extrait de lierre | 2 % |
| SEPICIDE™ HP | 1 % |
| Eau | q.s.p. 100 % |

### Exemple 27 : Crème confort pour peaux sensibles

| | |
|---|---|
| Composition A | 3% |
| SEPIGEL™ 305 | 2% |
| LIPACIDE™ C8G | 0,5% |
| MONTANOV™ 202 | 0,3% |
| Phytosqualan | 5% |
| Cyclométhicone/polymethyl cyclosiloxane | 10% |
| SEPICIDE™ HB | 0,2% |
| Eau | q.s.p. 100 % |

### Exemple 28 : Soin apaisant

| | |
|---|---|
| Composition A | 3% |
| SEPIGEL™ 305 | 0,7% |
| SIMULSOL™ 165 | 5% |
| capric caprylic triglyceride | 5% |
| SEPICIDE™ HB | 0,3% |
| SEPICIDE™ Cl | 0,2% |
| Eau | q.s.p. 100 % |

### Exemple 29 : Gel crème pour peaux sensibles

| | |
|---|---|
| Composition A | 3% |
| SIMULGEL™ 600 | 2% |
| Isohexadécane | 5% |
| huile de bourrache | 1% |
| SEPICIDE™ HB | 0,3% |
| SEPICIDE™ Cl | 0,2% |
| Parfum | 0,1% |
| Eau | q.s.p. 100 % |

### Exemple 30 : soin apaisant après soleil

| | |
|---|---|
| Composition A | 3% |
| SEPIGEL™ 502 | 4% |
| cyclométhicone et diméthiconol | 5% |
| LANOL™ 189 | 5% |
| SEPICIDE™ HB | 0,3% |
| SEPICIDE™ Cl | 0,2% |
| Parfum | 0,1% |
| Eau | q.s.p. 100 % |

### Exemple 31 : Gel apaisant pour les mains

| | |
|---|---|
| Composition A | 3% |
| SEPIGEL™ 305 | 4% |
| Isostéaryl isostéarate | 5% |
| MICROPEARL™ M305 | 1% |
| Glycérine | 10% |
| SEPICIDE™ HB | 0,3% |
| SEPICIDE™ Cl | 0,2% |
| Parfum | 0,2% |
| Eau | q.s.p. 100 % |

### Exemple 32 : Crème hydratante pour peaux sensibles

| | |
|---|---|
| MONTANOV™ 68 | 7% |
| Isostéaryl isostéarate | 5% |
| Diméthicone | 10% |
| Eau | q.s.p.100% |
| Composition A | 2% |
| SEPIGEL™ 305 | 1% |
| SEPICIDE™ HB | 0,3% |
| SEPICIDE™ Cl | 0,2% |
| Parfum | 0,2% |

### Exemple 33 : soin auto-bronzant

| | |
|---|---|
| Composition A | 3% |
| DHA | 1% |
| SEPIGEL™ 305 | 2% |
| MONTANOV™ 202 | 3% |
| Huile d'amande douce | 7% |
| Diméthicone | 3% |
| Parfum | 0,1% |
| SEPIFILM™ HB | 0,3% |
| SEPIFILM™ Cl | 0,2% |
| Eau | q.s.p.100% |

### Exemple 34 : Poudre compacte

| | |
|---|---|
| Talc | 57% |
| Polyméthylèneméthacrylate | 15% |
| Diméthicone | 15% |
| Stéarate de zinc | 5% |
| Isononylisononanoate | 5% |
| Composition A | 3% |
| Colorants | q.s. |

Les produits commerciaux cités ci-dessus ont les définitions suivantes :
Le MONTANOV™ 68 (cétéaryl glucoside), est une composition auto-émulsionnable, telle que décrite dans WO 92/06778, commercialisée par la société SEPPIC ;
Le SEPIGEL™ 502 est un agent épaississant à base de copolymères d'acrylamide, commercialisé par la société SEPPIC) ;
Le PEMULEN™ TR est un polymère acrylique, commercialisé par la société GOODRICH ;
Le MICROPEARL™ M 100 est une poudre ultra-fine au toucher très doux et à action matifiante, commercialisée par la société MATSUMO ;
Le SEPICIDE™ Cl ,imidazolin urée, est un agent conservateur commercialisé par la société SEPPIC ;
Le SIMULSOL™ 165 (stéarate de glycérol/stéarate de PEG100) est une composition auto-émulsionnable, commercialisée par la société SEPPIC ;
Le LANOL™ 1688 est un ester émollient à effet non gras commercialisé par la société SEPPIC ;
Le LANOL™ 14M et le LANOL™ S sont des facteurs de consistance commercialisés par la société SEPPIC ;
Le SEPICIDE™ HB ,(mélange de phénoxyéthanol/méthyl paraben /éthylparaben/ propylparaben/butylparaben), est un agent conservateur commercialisé par la société SEPPIC ;
Le MONTEINE™ CA est un agent hydratant commercialisé par la société SEPPIC ;
Le SCHERCEMOL™ OP est un ester émollient à effet non gras ; Le LANOL™ P est un additif à effet stabilisant commercialisé par la société SEPPIC ;
Le PARSOL™ MCX est de l'octyl paraméthoxycinnamate, commercialisé par la société GIVAUDAN ;
Le SEPIPERL™ N est un agent nacrant, commercialisé par la société SEPPIC, à base d'un mélange d'alkyl polyglucosides tels que ceux décrits dans WO 95/13863 ;
Le MICROPEARL™ SQL est un mélange de microparticules renfermant du squalane qui se libère sous l'action du massage; il est commercialisé par la société MATSUMO ;
Le LANOL™ 99 est de l'isononyl isononanoate, commercialisé par la société SEPPIC ;
le LANOL™ 37T est du triheptanoate de glycérol, commercialisé par la société SEPPIC ;
Le SOLAGUM™ L est un carraghénane commercialisé par la société SEPPIC,
Le MARCOL™ 82 est une huile paraffine commercialisée par la société ESSO;
Le LANOL™ 84D est du dioctylmalate commercialisé par la société SEPPIC ;
Le PARSOL NOX™ et l'EUSOLEX™ 4360 sont deux filtres solaires commercialisés respectivement par les sociétés GIVAUDAN et MERCK ;
Le DOW CORNING™ 245 FLuid est de la cyclométhicone, commercialisée par la société DOW CORNING ;
Le MONTANOV™ 202 (arachidyl glucoside/alcool béhenylique) est une composition auto-émulsionnable telle que décrite dans WO 92/06778, commercialisée par la société SEPPIC ;
Le LIPACIDE™ C8G (octanoyl glycine) est commercialisé par la société SEPPIC ;
Le SIMULGEL™ 600 est un agent épaississant à base de copolymères d'acrylamide commercialisé par la société SEPPIC ;
Le LANOL™ 189 est un ester de toucher riche et soyeux, commercialisé par la société SEPPIC ;
Le MICROPEARL™ M305 est un copolymère réticulé de polyméthyl méthacrylate est commercialisé par la société MATSUMO ;

## Revendications

1. Utilisation d'au moins un composé de formule (I): 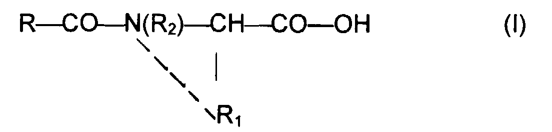 dans laquelle R représente la chaîne caractérisante d'un acide gras, saturé ou insaturé, linéaire ou ramifié, comportant de 3 à 30 atomes de carbone, R₁ ou 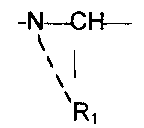 représente la chaîne ou le radical cyclique caractérisant d'un aminoacide, R₂ représente un atome d'hydrogène ou un radical alkyle comportant de 1 à 3 atomes de carbone, ou de leurs sels topiquement acceptables comme agent de texture, étant entendu que l'aminoacide caractérisé par R₁, n'est, ni la lysine, ni l'hydroxylysine, ni l'arginine.

2. Utilisation telle que définie à la revendication 1, pour laquelle, dans la formule (I), le fragment R―CO comporte de 7 à 22 atomes de carbone et représente notamment l'un des radicaux hexanoyle, heptanoyle, octanoyle (capryloyle), décanoyle (caproyle), undécylènoyle, dodécanoyle (lauroyle), tétradécanoyle (myristyle), hexadécanoyle (palmitoyle), octadécanoyle (stéaryle), eicosanoyle (arachidoyle), docosanoyle (behènoyle), octodécènoyle (oléyle), éicosènoyle (gadoloyle), docosènoyle (érucyle), octadécadiènoyle (linolènoyle).

3. Utilisation telle que définie à l'une des revendication 1 ou 2, pour laquelle, dans la formule (I), le fragment R-CO comporte de 12 à 22 atomes de carbone et plus particulièrement de 16 à 22 atomes de carbone.

4. Utilisation telle que définie à l'une des revendication 1 à 3, pour laquelle, dans la formule (I), la chaîne ou le radical cyclique caractérisant l'aminoacide, sont choisis parmi ceux de la glycine, de l'alanine, de la sérine, de l'acide aspartique, de l'acide glutamique, de la valine, de la thréonine, de la proline, de la leucine, de la phénylalanine, de l'isoleucine, de l'histidine, de la tyrosine, du tryptophane, de l'asparagine, de la cystéine, de la cystine, de la méthionine, de l'hydroxyproline, de l'ornithine et de la sarcosine.

5. Utilisation telle que définie à l'une des revendication 1 à 4, pour laquelle, dans la formule (I), la chaîne ou le radical cyclique caractérisant l'aminoacide sont choisis parmi ceux de la glycine, de la proline, de l'acide glutamique ou de la sarcosine.

6. Utilisation telle que définie à la revendication 5, pour laquelle la composition comprend au moins un composé de formule (la) correspondant à la formule (I), dans laquelle le radical cyclique divalent est celui de la proline, au moins un composé de formule (Ib) correspondant à la formule (I) dans laquelle la chaîne caractérisante est celle de l'acide glutamique et éventuellement au moins un composé de formule (Ic), correspondant à la formule (I) dans laquelle R₂ représente un radical méthyle et R₁ la chaîne caractérisante de la sarcosine.

7. Utilisation telle que définie à l'une des revendication 1 à 6, d'une composition comprenant soit de la N-palmitoyl proline et de d'acide N-palmitoyl glutamique ou leurs sels de sodium ou de magnésium soit de la N-palmitoyl proline, de l'acide N-palmitoyl glutamique et de N-palmitoyl sarcosine ou leurs sels de sodium ou de magésium.

8. Utilisation telle que définie à l'une des revendication 1 à 5, d'une composition comprenant de la N-cocoyl glycine.

9. Composition (A) comprenant :
a) De 10% à 95%, et plus particulièrement, de 60% à 80% en poids d'au moins un composé de formule (I): 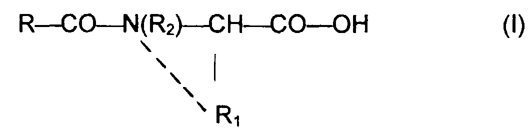 dans laquelle R représente la chaîne caractérisante d'un acide gras, saturé ou insaturé, linéaire ou ramifié, comportant de 12 à 22 atomes de carbone, R₁ ou 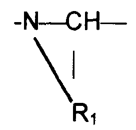 représente la chaîne ou le radical cyclique caractérisant d'un aminoacide, R₂ représente un atome d'hydrogène ou un radical alkyle comportant de 1 à 3 atomes de carbone, étant entendu que l'aminoacide caractérisé par R₁ n'est ni la lysine, ni l'hydroxylysine, ni l'arginine, ou un de leurs sels topiquement acceptables, et,
b) de 90% à 5% et plus particulièrement de 20% à 40% en poids d'au moins un composé de formule (II)
R-CO-OH (II),
ou un de leurs sels topiquement acceptables.

10. Composition (A) telle que définie à la revendication 9, pour laquelle le radical R comporte de 16 à 22 atomes de carbone, et plus particulièrement de 16 à 18 atomes de carbone.

11. Composition (A) telle que définie à la revendication 10, comprenant de 30% à 50% en poids de palmitoyl proline, de 5 à 25% en poids d'acide N-palmitoyl glutamique, de 0% à 25% en poids de N-palmitoyl sarcosine et d' environ 5% à environ 40% en poids d'acide palmitique.

12. Composition (A) telle que définie à la revendication 9, comprenant de 30% à 70% en poids de cocoyl glycine, de 0% à 25% en poids de N-cocoyl sarcosine et d' environ 5% à environ 45%, et plus particulièrement de 5% à environ 20% en poids du mélange d'acides gras issu de l'huile de coprah.

13. Formulation cosmétique, dermopharmaceutique ou pharmaceutique comprenant comme agent de texture, une quantité efficace d'au moins un composé de formule (I) tel que définie à l'une des revendications 1 à 8.

14. Utilisation d'une composition (A) telle que définie à l'une des revendications 9 à 12, pour préparer une formulation cosmétique, dermopharmaceutique ou pharmaceutique
